# FASCICULE DE BREVET EUROPEEN

(11) **EP 2 591 816 B1**
(45) Date de publication et mention de la délivrance du brevet: **13.01.2021**
(21) Numéro de dépôt: 12192004.5
(22) Date de dépôt: 09.11.2012
(51) Int. Cl.: A61M 5/32

(54) **Vecteur d'injection d'un produit viscoélastique de comblement**
Vektor zum Injizieren eines viskoelastischen Füllprodukts
Carrier for injecting a viscoelastic filling product

(30) Priorité: 10.11.2011 FR 1160239
(43) Date de publication de la demande: 15.05.2013
(73) Titulaire: Persat, Jean-Charles, 1239 Canton de Geneve (CH)
(72) Inventeur: Persat, Jean-Charles, 1239 Canton de Geneve (CH)
(74) Mandataire: Cabinet Beau de Loménie

(56) Documents cités:
- EP-A1- 2 455 115
- EP-A2- 1 787 592
- WO-A1-2008/055001
- WO-A1-2008/155145
- FR-A1- 2 942 410
- FR-A1- 2 942 410

## Description

La présente invention concerne le domaine technique général des vecteurs assurant une injection de manière non invasive, d'un produit viscoélastique de comblement dans le corps humain et elle vise plus précisément les vecteurs d'injection de produits viscoélastiques de comblement tels que par exemple d'une part, les produits de remplissage utilisés dans le domaine de la chirurgie esthétique non invasive par voie transcutanée et d'autre part, les produits de cémentoplastie ayant une activité thérapeutique et reconstructive.

L'objet de l'invention trouve une première application particulièrement avantageuse dans le domaine de la chirurgie esthétique ou réparatrice consistant à réparer des défauts esthétiques cutanés, du regard, de la silhouette, du contour du visage et du corps en général. La technique de traitement consiste à réduire les défauts esthétiques par comblement des différentes couches cutanées, sous cutanées, musculaires et péri-osseuses, soit par la greffe autologue des propres adipocytes du patient (cellules graisseuses prélevées sur les sites anatomiques les plus riches en graisse), soit par des substances biologiques issues des techniques de thérapie cellulaire, soit par l'injection d'un produit de comblement de synthèse tel que l'acide hyaluronique qui se présente sous la forme d'un gel hydrophile avec différents niveaux de viscosité.

L'objet de l'invention trouve une autre application particulièrement avantageuse pour les traitements de cémentoplastie visant à disperser les produits en phase pâteuse tels que des ciments ou des substituts osseux favorisant la repousse de l'os, au sein des foyers tissulaires à traiter tels que tumeurs, ostéoporoses, fractures et dégénérescences. Cette application vise particulièrement la cémentoplastie du rachis, des os longs et des os plats.

Dans l'état de la technique, il est connu d'assurer l'injection d'un produit viscoélastique de comblement à l'aide de vecteurs d'injection tels que des canules ou aiguilles permettant de traverser les tissus mous (peau, muscles, tendons, graisses) ou des trocarts destinés à une utilisation sur les os.

D'une manière générale, un vecteur d'injection se présente sous la forme d'un tube présentant une ou plusieurs ouvertures de passage pour le produit viscoélastique de comblement. La taille des orifices de passage est adaptée de manière à diminuer la résistance au passage des produits viscoélastique de comblement.

Ainsi, la demande de brevet FR 2 942 410 décrit un vecteur d'injection d'un produit viscoélastique ou de comblement comportant une aiguille tubulaire semi-rigide d'axe longitudinal présentant une extrémité distale et une extrémité proximale de raccordement à un dispositif d'injection. Cette aiguille présente une face interne tubulaire avec un diamètre interne et une face externe tubulaire délimitant une section droite transversale externe. Une ouverture latérale de sortie est aménagée dans l'aiguille à proximité de l'extrémité distale.

L'ouverture latérale de sortie possède un bord périphérique avec un profil arrondi pour être en particulier atraumatique.

Le document EP 1 787 592 décrit un vecteur d'injection de forme incurvée présentant des orifices de sortie de forme elliptique.

Le document WO 2008/155145 décrit un cathéter comportant un tube flexible dans lequel sont aménagés des orifices avec des indentations. Un tel cathéter qui vise des applications de drainage n'est pas adapté pour l'injection d'un produit viscoélastique.

Dans le même sens, la demande de brevet EP 2 455 115 qui a une date de publication postérieure à la présente demande concerne un vecteur de prélèvement possédant des orifices avec des arêtes coupantes. Un tel vecteur n'est pas adapté à l'injection d'un produit viscoélastique.

La demande de brevet WO 2008/055001 décrit selon une variante de réalisation, une aiguille d'injection comportant une unique ouverture de forme sensiblement ovale.

D'une manière générale, il a été constaté que le produit viscoélastique de comblement progressait en masse compacte autour de l'orifice de sortie des vecteurs d'injection de l'art antérieur, en faisant un amas autour de cette ouverture de sortie. Il s'ensuit une mauvaise répartition du produit viscoélastique de comblement.

Par ailleurs, les tissus situés dans l'environnement de l'ouverture de sortie du vecteur d'injection agissent, de par leur viscosité, comme un clapet sur cette ouverture de sortie en créant une contre pression gênant la sortie du produit viscoélastique de comblement. Cette contre pression agit aussi dans le sens d'un déplacement en masse compacte du produit viscoélastique de comblement, ce qui privilégie le chemin de moindre résistance autour de l'ouverture d'injection.

La présente invention vise à remédier aux inconvénients de l'état de la technique en proposant un vecteur d'injection conçu pour assurer la dispersion dans la profondeur des tissus, du produit viscoélastique de comblement, cette dispersion étant réalisée de manière plus homogène par rapport aux techniques connues.

Pour atteindre un tel objectif, l'objet de l'invention concerne un vecteur d'injection d'un produit viscoélastique ou de comblement conforme à la revendication 1.

Le vecteur d'injection selon l'invention est donc conçu pour éviter l'agglomération du produit viscoélastique de comblement à la sortie de l'ouverture de passage du vecteur d'injection.

De plus, le vecteur d'injection selon l'invention peut présenter en outre en combinaison au moins l'une et/ou l'autre des caractéristiques additionnelles suivantes :
- la fente possède une largeur inférieure ou égale à 0,2 mm et supérieure ou égale à 0,01 mm, pour une première catégorie de vecteurs,
- la fente possède une largeur inférieure ou égale à 2 mm et supérieure ou égale à 0,01 mm, pour une deuxième catégorie de vecteurs,
- la fente possède une hauteur comprise entre 2 mm et 0,08 mm.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective d'un exemple de réalisation d'un vecteur d'injection conforme à l'invention.
La **Figure 2** est une vue latérale du vecteur d'injection conforme à l'invention.
La **Figure 3** est une vue en coupe prise selon les lignes **A-A** de la **Fig. 2****.**
La **Figure 4** est une vue en coupe partielle prise sensiblement selon les lignes **B-B** de la **Fig. 3****.**
La **Figure 5** est un schéma illustrant la variation de la vitesse de circulation du produit viscoélastique à l'intérieur du vecteur d'injection en fonction de la longueur du vecteur pris en considération selon son axe longitudinal **xx'.**

Tel que cela ressort des **Figures,** l'objet de l'invention concerne un vecteur d'injection **1** tel qu'une canule ou une aiguille au sens général adapté pour assurer l'injection dans le corps humain d'un produit viscoélastique de comblement. Par exemple, dans le domaine de la chirurgie esthétique ou réparatrice, le vecteur d'injection **1** permet d'injecter pour la microplastie, de l'acide hyaluronique ou toute autre substance de remplissage et pour la liporeconstruction, de la graisse autologue. Par exemple, dans le domaine de la cémentoplastie du rachis, des os longs et des os plats, le vecteur d'injection **1** permet d'injecter des ciments ou du substitut osseux favorisant la repousse de l'os.

Ce vecteur d'injection **1** comporte une aiguille tubulaire **2** semi-rigide présentant un axe longitudinal de symétrie **xx'.** Avantageusement, cette aiguille tubulaire **2** est réalisée en un matériau métallique tel en acier inoxydable. Cette aiguille tubulaire **2** présente une extrémité proximale **3** de raccordement par un embout d'adaptation non représenté, à un dispositif d'injection non représenté également tel qu'une seringue, ou un injecteur pour produit de haute viscosité.

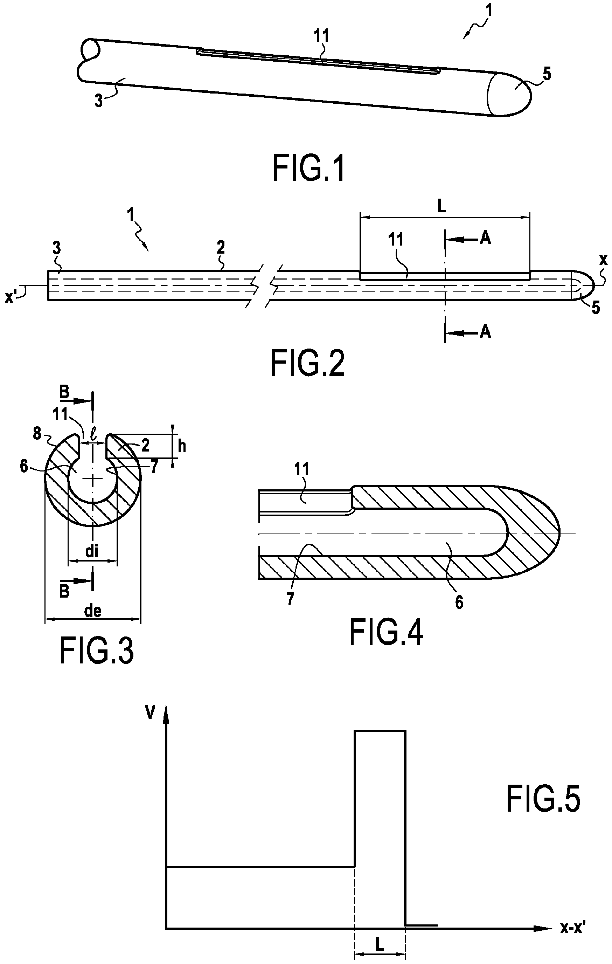

Cette aiguille tubulaire **2** comporte à l'opposé de l'extrémité proximale **3,** une extrémité distale **5** fermée qui dans l'exemple illustré à la **Fig. 4** est arrondie ou hémisphérique. L'aiguille tubulaire **2** présente ainsi une extrémité fermée pouvant être dite mousse atraumatique ou être biseautée ou affutée.

L'aiguille tubulaire **2** présente un canal interne **6** délimité par une face interne tubulaire **7** présentant par rapport à l'axe longitudinal **x, x'** une section droite transversale interne circulaire de diamètre **di.** Cette aiguille tubulaire **2** présente une face externe tubulaire **8** délimitant par rapport à l'axe longitudinal **x, x'** une section droite transversale externe circulaire possédant un diamètre externe **de (****Fig. 3****).**

L'aiguille tubulaire **2** présente selon cette variante de réalisation, une paroi d'épaisseur constante sur toute sa longueur tel que cela ressort des dessins, et une ouverture latérale de sortie **11** est aménagée dans l'aiguille tubulaire **2** à proximité de l'extrémité distale **5.** Cette ouverture de sortie **11** débouche ainsi sur les faces interne **7** et externe **8** de l'aiguille tubulaire **2** permettant ainsi une communication entre le canal interne **6** et l'extérieur de l'aiguille. Cette ouverture de sortie **11** assure ainsi la sortie du produit viscoélastique de comblement provenant du canal interne **6** et introduit à l'aide d'un dispositif d'injection non représenté, tel qu'une seringue munie d'un piston ou un injecteur pour produit à haute viscosité.

Conformément à l'invention, cette ouverture latérale de sortie **11** est une fente s'étendant parallèlement à une génératrice de l'aiguille c'est-à-dire parallèlement à l'axe longitudinal de symétrie **xx'** de l'aiguille. L'ouverture de sortie **11** présente une largeur **l** sensiblement constante sur toute sa longueur **L** prise selon l'axe de symétrie longitudinale **xx'.**

Ainsi, tel que cela ressort clairement des **Figures,** les deux bords longitudinaux de la fente **11** s'étendent parallèlement entre eux et parallèlement à une génératrice de l'aiguille. Il est à noter que la génératrice de l'aiguille est droite ou rectiligne puisque l'aiguille n'est pas cintrée ou courbée. Ainsi, la fente **11** s'étend de manière rectiligne ou droite.

La largeur **l** de l'ouverture de sortie **11** est inférieure ou égale à 0,5 fois le diamètre interne **di** de l'aiguille et supérieure ou égal à 0,2 fois le diamètre interne **di** l'aiguille. L'ouverture de sortie **11** possède une longueur **L** prise le long d'une génératrice inférieure ou égale à 4 fois le diamètre interne **di** de l'aiguille et supérieure ou égale à 1 fois le diamètre interne de l'aiguille.

Une telle ouverture de sortie **11** permet de délimiter à l'intérieur du canal **6,** une chambre de compression située en amont par rapport à l'ouverture de sortie **11.** En effet, la réalisation d'une unique fente **11** avec une section de passage réduite par rapport à la section interne du tube conduit à une variation du rapport de la pression sur la vitesse du produit viscoélastique de comblement permettant d'augmenter la vitesse et la pression du produit viscoélastique de comblement en sortie de l'ouverture de sortie **11.** Ainsi, l'augmentation de la résistance au passage du produit viscoélastique de comblement conduit à une dispersion en profondeur du produit viscoélastique de comblement en sortie de l'ouverture **11.**

Selon une première variante de réalisation, le vecteur d'injection **1** est destiné à la chirurgie esthétique ou réparatrice. Selon cette variante, l'ouverture de sortie **11** possède une largeur **l** inférieure ou égale à 0,2mm et supérieure ou égale à 0,01 mm.

Dans le cas de l'utilisation du vecteur d'injection selon l'invention pour le domaine de la cémentoplastie, l'ouverture de sortie **11** du vecteur d'injection possède une largeur **l** inférieure ou égale à 2 mm et supérieure ou égale à 0,01 mm.

Selon une caractéristique de l'invention, la hauteur **h** de l'ouverture de sortie **11** prise dans le plan de symétrie entre les faces interne **7** et externe **8,** est comprise entre 2 mm et 0,08 mm. Pour certaines applications, la hauteur **h** de l'ouverture de sortie **11** peut atteindre 0,01 mm.

Le vecteur d'injection **1** selon l'invention permet de réaliser l'injection du produit viscoélastique sous la forme d'une nappe s'établissant transversalement par rapport à l'axe du vecteur. A cet effet, le vecteur d'injection **1** selon l'invention est destiné à équiper un dispositif d'injection tel qu'une seringue munie d'un piston ou d'un injecteur pour produit à haute viscosité. L'injection du produit viscoélastique est obtenue par le déplacement du piston de la seringue permettant la compression du produit viscoélastique introduit préalablement à l'intérieur de la seringue.

Compte tenu des caractéristiques de la fente **11** par rapport à l'aiguille **2,** le produit viscoélastique est acheminé en amont de la fente **11,** dans une chambre de compression pour ensuite être projeté à travers la fente **11** selon un pic de vitesse comme illustré à la **Fig. 5****.** Il ressort clairement de cette **Fig. 5** que la vitesse du produit viscoélastique connaît un pic à l'endroit de l'ouverture de sortie **11,** selon toute sa longueur **L.**

L'invention n'est pas limitée aux exemples décrits et représentés car diverses modifications peuvent y être apportées sans sortir de son cadre.

## Revendications

1. Vecteur d'injection d'un produit viscoélastique ou de comblement comportant une aiguille tubulaire **(2)** semi-rigide d'axe longitudinal présentant une extrémité distale fermée **(5)** et une extrémité proximale **(3)** de raccordement à un dispositif d'injection, cette aiguille présentant un canal interne **(6)** délimité par une face interne tubulaire **(7)** avec un diamètre interne **(Si)** et une face externe tubulaire **(8)** délimitant une section droite transversale externe **(Se),** une ouverture latérale de sortie (**11**) étant aménagée dans l'aiguille à proximité de l'extrémité distale et présentant d'une part une longueur **L** prise le long d'une génératrice, inférieure ou égale à quatre fois le diamètre interne **(di)** de l'aiguille et supérieure ou égale à une fois le diamètre interne **(di)** de l'aiguille et d'autre part, une largeur **l** supérieure ou égale à 0,2 fois le diamètre interne **(di), caractérisé en ce que** l'ouverture latérale de sortie **(11)** délimite à l'intérieur du canal interne **(6),** une chambre de compression située en amont par rapport à l'ouverture latérale de sortie **(11)** et **en ce que** l'ouverture latérale de sortie **(11)** est une fente comportant deux bords longitudinaux s'étendant parallèlement entre eux et à une génératrice de l'aiguille en présentant une largeur **l** sensiblement constante, inférieure ou égale à 0,5 fois le diamètre interne **(di)** de l'aiguille de manière à délimiter à l'intérieur de l'aiguille en regard de la fente, une chambre de compression pour assurer la projection du produit viscoélastique à travers la fente.

2. Vecteur d'injection selon la revendication 1, **caractérisé en ce que** la fente **(11)** possède une largeur **l** inférieure ou égale à 0,2 mm et supérieure ou égale à 0,01 mm, pour une première catégorie de vecteurs.

3. Vecteur d'injection selon la revendication 1, **caractérisé en ce que** la fente **(11)** possède une largeur **l** inférieure ou égale à 2 mm et supérieure ou égale à 0,01 mm, pour une deuxième catégorie de vecteurs.

4. Vecteur d'injection selon la revendication 1, **caractérisé en ce que** la fente **(11)** possède une hauteur **(h)** comprise entre 2 mm et 0,08 mm.

## Patentansprüche

1. Vektor zum Injizieren eines viskoelastischen Produkts oder eines Füllprodukts, umfassend eine halbstarre Hohlnadel (2) mit einer Längsachse, die ein geschlossenes distales Ende (5) und ein proximales Ende (3) mit Anschluss an eine Injektionsvorrichtung aufweist, wobei diese Nadel einen Innenkanal (6) aufweist, der durch eine röhrenförmige Innenfläche (7) mit einem Innendurchmesser (Si) und eine röhrenförmige Außenfläche (8) begrenzt ist, die einen äußeren geraden Querschnitt (Se) begrenzt, wobei eine seitliche Ausgangsöffnung (11) in der Nadel in der Nähe des distalen Endes angeordnet ist und einerseits eine Länge L entlang einer Mantellinie, die kleiner als das oder gleich dem Vierfachen des Innendurchmessers (di) der Nadel und größer als das oder gleich dem Einfachen des Innendurchmessers (di) der Nadel ist, und andererseits eine Breite I aufweist, die größer als das oder gleich dem 0,2-Fachen des Innendurchmessers (di) ist, **dadurch gekennzeichnet, dass** die seitliche Ausgangsöffnung (11) im Inneren des Innenkanals (6) eine Kompressionskammer begrenzt, die sich stromaufwärts in Bezug auf die seitliche Ausgangsöffnung (11) befindet, und dass die seitliche Ausgangsöffnung (11) ein Schlitz ist, der zwei Längskanten umfasst, die sich parallel zueinander und zu einer Mantellinie der Nadel erstrecken und eine im Wesentlichen konstante Breite l aufweisen, die kleiner als das oder gleich dem 0,5-Fachen des Innendurchmessers (di) der Nadel ist, um im Inneren der Nadel in Bezug auf den Schlitz eine Kompressionskammer zu begrenzen, um die Projektion des viskoelastischen Produkts durch den Schlitz sicherzustellen.

2. Vektor zum Injizieren nach Anspruch 1, **dadurch gekennzeichnet, dass** der Schlitz (11) eine Breite l, die kleiner als oder gleich 0,2 mm und größer als oder gleich 0,01 mm ist, für eine erste Vektorkategorie besitzt.

3. Vektor zum Injizieren nach Anspruch 1, **dadurch gekennzeichnet, dass** der Schlitz (11) eine Breite l, die kleiner als oder gleich 2 mm und größer als oder gleich 0,01 mm ist, für eine zweite Vektorkategorie besitzt.

4. Vektor zum Injizieren nach Anspruch 1, **dadurch gekennzeichnet, dass** der Schlitz (11) eine Höhe (h) besitzt, die zwischen 2 mm und 0,08 mm beträgt.

## Claims

1. A vector for injecting a viscoelastic or filling product including a semi-rigid tubular needle **(2)** of longitudinal axis having a closed distal end **(5)** and a proximal end **(3)** for connection to an injection device, this needle having an inner channel **(6)** delimited by a tubular inner face **(7)** with an internal diameter **(Si)** and a tubular outer face **(8)** delimiting an outer transverse cross-section **(Se),** a lateral outlet opening **(11)** being arranged in the needle in the vicinity of the distal end and having on the one hand a length **L** taken along a generatrix, smaller than or equal to four times the internal diameter **(di)** of the needle and greater than or equal to once the internal diameter **(di)** of the needle and on the other hand, a width **l** greater than or equal to 0.2 times the internal diameter **(di), characterized in that** the lateral outlet opening **(11)** delimits inside the inner channel **(6),** a compression chamber located upstream with respect to the lateral outlet opening **(11)** and **in that** the lateral outlet opening **(11)** is a slot including two longitudinal edges extending parallel to each other and to a generatrix of the needle by having a substantially constant width **l**, smaller than or equal to 0.5 times the internal diameter **(di)** of the needle so as to delimit inside the needle facing the slot, a compression chamber to ensure the projection of the viscoelastic product through the slot

2. The injection vector according to claim 1, **characterized in that** the slot **(11)** has a width **l** smaller than or equal to 0.2 mm and greater than or equal to 0.01 mm, for a first category of vectors.

3. The injection vector according to claim 1, **characterized in that** the slot **(11)** has a width **l** smaller than or equal to 2 mm and greater than or equal to 0.01 mm, for a second category of vectors.

4. The injection vector according to claim 1, **characterized in that** the slot **(11)** has a height **(h)** comprised between 2 mm and 0.08 mm.
